# EUROPEAN PATENT APPLICATION

(11) **EP 4 053 293 A1**
(43) Date of publication of application: **07.09.2022**
(21) Application number: 21160578.7
(22) Date of filing: 03.03.2021
(51) Int. Cl.: C12Q 1/6844

(54) **PROTECTED ISOTHERMAL NUCLEIC ACID AMPLIFICATION (PINA) METHODS FOR POINT-OF-NEED DIAGNOSIS OF EMERGING INFECTIOUS DISEASES**

(71) Applicant: midge medical GmbH, 12103 Berlin (DE)
(72) Inventor: RIESTER, Markus, Wiesbaden (DE); WEIDMANN, Manfred, Berlin (DE)
(74) Representative: Eisenführ Speiser

(57) **Abstract**

The present invention relates to a fast and reliable method for protected isothermal nucleic acids amplification (PINA) of DNA and RNA. Particularly, the invention relates to diagnostic methods for rapidly diagnosing, for example, an infectious agent, in a biological probe of interest. Particularly, the methods are suitable to be performed on a handheld and portable diagnostic system for performing the amplification method in a non-laboratory environment ("home-test").

## Description

### Technical Field

The present invention relates to a fast and reliable method for protected isothermal nucleic acids amplification (PINA) of DNA and RNA. Particularly, the invention relates to diagnostic methods for rapidly diagnosing, for example, an infectious agent, in a biological probe of interest. Particularly, the methods are suitable to be performed on a handheld and portable diagnostic system for performing the amplification method in a non-laboratory environment ("home-test").

### Background of the Invention

The SARS-CoV-2 virus outbreak declared as a world pandemic by the WHO in March 2020 quite strikingly demonstrated the need for reliable and fast diagnostics, particularly for newly emerged infectious diseases for which no prophylactic vaccine or curative medicine is available. Hitherto, sensitive and fast diagnostic tools reliably identifying the SARS-CoV-2 virus in a short time are not yet available. This is highly problematic as there is a significant time gap between testing and the availability of the test result so that an isolation of infected individuals can often only be initiated after a further virus spread could take place.

Generally, several antigen-based immunoassays and PCR-based techniques are presently used to diagnose a potential SARS-CoV-2 infection on a protein and on a nucleic acid level, respectively. Sensitivity, specificity and speed (time to result) are of utmost importance for a reliable diagnostic assay or kit.

Regarding nucleic acid-based preparative, cloning and diagnostic techniques, the development of polymerase chain reaction (PCR) in the 1980^{ies} by the later Nobel laureate Kary Mullis and his team as a rapid and reliable method to amplify DNA revolutionized molecular biology in general, as scientists suddenly had the opportunity to obtain millions of copies of a DNA target molecule of interest in short time. Simplicity and efficiency (e.g., possibilities nowadays to perform single-molecule/cell PCR) represent significant advantages of PCR techniques.

Nucleic acid detecting techniques like molecular real-time PCR assays are usually very sensitive, and offer target specificity but they still suffer from certain drawbacks. In a PCR the primer annealing can only occur through repetitive cycling between high temperatures allowing thermal denaturing of double-stranded DNA (dsDNA) and lower temperatures that allow annealing of primers, and primer extension by the polymerase. The inherent need for ongoing cycles of rapid cooling and heating (twenty to fifty cycles of two to three alternating temperatures), accurate temperature control, and the use of high (>90°C) temperatures requires specialized thermal cyclers and is highly energy-consuming. Thus, PCR-based methods for DNA amplification have the huge disadvantage to require highly equipped laboratories and well-trained personnel. Further, the highest possible efficiency for PCR methods is inevitably limited to one doubling per cycle, which does not allow quick (10 - 20 min) amplification while maintaining a high yield. The outbreak of SARS-CoV-2 has shown that PCR-based diagnosis by specialized laboratories is too slow and cumbersome for a responsive monitoring of infectious events. Thus, it is still impossible to react quickly to local virus outbreaks and to stop the viral spread effectively. It was tried to meet the evident requirement for fast and easy testing and point-of-need diagnosis by antigen-based diagnostic tools. However, the detection of viral RNA or DNA, which is critical for the accurate diagnosis of a viral infection, remains to be slow and cumbersome. Therefore, new portable diagnostic solutions having a high sensitivity and specificity and being able to provide reliable results at the place of testing are urgently needed.

A solution that allows both quick point-of-need diagnosis and the detection of viral RNA or DNA might be PCR alternatives, the so-called isothermal amplification methods (for a review, see Zanoli and Spoto, Biosensors (Basel), 2013 3(1): 18-43)). The huge advantage over PCR is the fact that isothermal nucleic acid amplification methods are not requiring any thermal cycling at all, but can rather be conducted at constant temperatures. This makes the amplification process much easier to operate and to control and less energy-consuming than PCR methods. The constant temperature of isothermal methods additionally allows the use of fully enclosed micro-structured devices, which reduces the risk of sample contamination and implies low sample consumption, multiplex DNA analysis, and portable devices realization. Finally, the constant temperature would be highly preferably for point-of-need and/or portable diagnostic devices, as recently developed by the present applicant (DE 10 2020 109 744.1).

There are numerous isothermal amplification strategies available at date, including nucleic acid sequence-based amplification (NASBA), loop-mediated isothermal amplification (LAMP), helicase-dependent amplification (HDA), rolling circle amplification (RCA), multiple displacement amplification (MDA), recombinase polymerase amplification (RPA), and nicking enzyme amplification reaction (NEAR/EXPAR) (see: Zanoli and Spoto, 2013, Biosensors (Basel). 2013 Mar; 3(1): 18-43; for a comprehensive survey on nowadays RPA techniques: Li et al., Analyst, 2019, 144, 31, pp. 31 to 67, or strand invasion based amplification (SIBA^{®}- SIBA in the following) Hoser et al., 2014 PLoS ONE 9(11): e112656, or NEAR/EXPAR: Van Ness et al., 2003, PNAS April 15, 2003 100 (8) 4504-4509). What the various isothermal amplification strategies have in common is that they all offer particular methods for allowing the initiation of the polymerase reaction. In conventional PCR this is achieved through the thermal cycling, which allows the primer to anneal to single stranded DNA (ssDNA) after denaturing.

LAMP uses primers that allow the formation of hairpins, in combination with a displacing DNA polymerase to generate exposed ssDNA to which further primer molecules can anneal (Notomi et al., 2000, Nucleic Acids Research 28: e63). However, apart from the challenging primer design, there is also the risk that the use of numerous large primers, as required, may result in false-positive results - an outcome definitely to be avoided during diagnostic applications, particularly for diagnosing infectious diseases, where a false-positive result may be highly detrimental. Further, there is the problem that LAMP reactions require high temperatures not as such practicable for portable devices, particularly diagnostic devices for use in a home environment, due to regulatory and practical issues. In General, real-time (RT)-LAMP assays have a run time of about 30 minutes and need devices to maintain an amplification temperature of 65°C. Consequently, LAMP will not be the method of choice for quick and reliable diagnostic nucleic acid amplifications results in a handheld device, which has to fulfill certain regulatory requirements to be suitable for non-laboratory use.

RPA and SIBA technology both rely on the use of a recombinase during the binding and amplification process. Initially, a nucleoprotein complex constituted by oligonucleotide primers and the recombinase proteins is formed for RPA- and SIBA-type nucleic acid amplification strategies. These complexes facilitate the primer binding to the template DNA (Boyle et al., 2013, doi:10.1128/mBio.00135-13; Daher et al., 2014, DOI: 10.1016/j.mcp.2014.11.005).

In parallel to the biochemical advancements in nucleic acid amplification, also the development of devices including suitable reaction and detection chambers for performing nucleic acid amplification in an optimized manner have steadily developed. A great trend has been the development of microfluidic devices and, in general, a trend of miniaturizing or "nanotizing reaction chambers", i.e., putting them into nanoforms, has been of great interest to decrease the sample volumes and thus the amount of reagents needed and to achieve advantages in biochemical detection, time to result, faster thermal transfer, and to have the potential for automation and integration.

As clearly exemplified during the Corona pandemic in 2020 - 2021, there is still an ongoing global need for providing fast and reliable nucleic acid amplification techniques, which cannot exclusively be performed in specialized diagnostic centers, but can rather be performed immediately at the site, where a biological sample is obtained from a person to be tested. Further, it has been observed at a global scale that time to result is of paramount importance as, unless strict and prophylactic quarantine rules apply, there is a huge risk that an infected yet symptomless patient may spread an infectious disease until receiving a (reliable) test result, which can currently last anything from 24-36 hours or more.

In light of the need for quick point-of-need diagnostics, RPA strategies have the great disadvantage that they rely on the recombinase as the pivotal element for primer binding. Unfortunately, the recombinase additionally requires at a recombinase loading factor and a single-strand binding (SSB) protein. As the recombinase requires energy for its activity, it further requires adenosine triphosphate (ATP) and an energy regeneration system consisting of phosphocreatine and an additional enzyme, creatine kinase. Thus, RPA requires a multitude of different Enzymes, proteins and other co-factors, making it a highly complex and cost-intensive reaction approach and, therefore, is not method of choice either for quick diagnostic tool that can be widely used at the point of need.

This is especially true if the diagnosis tool also aims to detect RNA, which is a great benefit as this allows the direct detection of nucleic acid material from RNA viruses and also the detection of any viral transcripts DNA viruses. If RNA is also to be detected, the already highly complex reaction mix requires even another enzyme, a reverse transcriptase. The reverse transcriptase rewrites RNA into homologous DNA sequences, which can then be detected by PCR or isothermal amplification methods.

Technically, it is very complex to substitute a naturally highly specialized enzyme like the recombinase, or to find solutions that can yield a desirable sensitivity and specificity while leaving out the critical component. However, it is an important task to reduce the complexity of diagnostic DNA amplification methods to make the diagnostic tool simpler and less error prone, particularly if not handled by trained experts but are rather used as home tests. Further, a reduced complexity, especially the reduction of required enzymes, reduces the costs of such a diagnostic tool, which is critical for monitoring of infectious events by quick and widespread diagnosis in large numbers.

It was thus an object of the present invention to provide a new isothermal nucleic amplification method suitable and specifically optimized for being conducted under non-laboratory conditions in a handheld or portable diagnostic device to enable point-of-need diagnostic infections even under a home environment and to provide fast and reliable diagnostic results for diagnosing a potential infection in a test sample directly applied to the diagnostic device. Particularly, it was an object to drastically reduce the complexity of the enzymatic components needed for the reaction to fit the needs of providing cheap, but still highly reliable and fast testing tools.

### Summary of the Invention

The present invention is as defined in the attached set of claims.

### Brief Description of Drawings

**Figure 1** (Fig. 1) Visualization of protected isothermal DNA amplification results using the Sars-CoV2 RdRP gene as target and RB69 GP32 and T4 GP32 as single-strand binding proteins.
**Figure 2** (Fig. 2) Comparison of an isothermal amplification of the Sars-CoV2 RdRP gene using T4 GP32 in an RPA and in a PINA approach.

### Detailed Description

Even though nucleic acid amplification techniques, including isothermal amplification strategies, have made enormous progress over the last decades, the crucial working enzymes for all of these techniques are usually not exchanged, or they are slightly modified in a stepwise manner.

In short, an established technique for isothermal DNA/RNA amplification of the prior art usually starts with the binding of a phage-derived T4 UvsX protein as central recombinase, assisted by the T4 UvsY acting as loading factor, to the primers. This complex invades the double-stranded (ds) DNA, forming a so-called D-loop in which the primer hybridizes with the template strand, initiating a strand exchange reaction. The unwound complementary strand is stabilized by a single-strand binding (SSB) protein, where usually a T4-derived gp32 protein is used. Primer incorporation allows a strand displacing DNA polymerase, usually a *Bsu* (*Bacillus subtilis-derived*) or *Sau* (*Staphylococcus aureus-derived*) polymerase, to initiate the synthesis from the free 3'-OH of the primer. As the polymerization continues, the two parental strands will thus continue to separate. Incorporation of both forward and reverse primers then enables strand synthesis to occur in both directions simultaneously, and ultimately results in the exponential accumulation of amplified duplex DNA, consisting of the sequence between the forward and reverse primers. Consequently, standard RPA requires a significant amount of protein/enzyme interaction partners to guarantee efficient DNA/RNA amplification.

It is a particular aim of the present invention to provide an isothermal amplification method that has a low complexity compared while allowing effective isothermal nucleic acid amplification, in order to provide diagnostic tools that are cheap and don't have to be used by trained professionals in a laboratory environment.

Thus, it is a particular advantage of the present invention that it does not require any recombinase. Without the need for a functional recombinase, also the need for any recombinase loading factor is rendered obsolete. Moreover, the Recombinase hydrolyzes ATP to perform its function, thus, without a recombinase, ATP and any energy regeneration system, typically comprising phosphocreatine and even an additional enzyme, creatine kinase, do not have to be added to the reaction mix. Therefore, in the methods according to the present invention the reaction additives and the number of different proteins and enzymes can be drastically reduced.

In a preferred embodiment, the method according to any aspect of the present invention proceeds without adding an energy regeneration enzyme.

An important feature of the present invention is its ability to reliably amplify nucleic acid sequences of any kind, particularly also including linear target molecules as shown in Example 2. For a method that is to be used as a diagnostic tool for viral infections, the suitability of the method to detect linear targets is essential. Different viruses contain genetic material in various different forms as linear or circular, double-stranded or single-stranded DNA or RNA. Thus, an isothermal amplification method for detecting viral nucleic acid material needs to be able to detect all possible various forms viral nucleic acid material. Detection of linear nucleic acid targets is necessary for viruses with linear DNA but also for all RNA viruses as the RNA has to be reverse transcribed into linear complementary DNA (cDNA). Further, the detection of linear nucleic acid targets is required to detect RNA transcripts, also from DNA viruses.

In one embodiment according to the first aspect of the present invention, there may be provided an isothermal method of nucleic acid amplification of at least one target sequence, preferably in a biological sample, the method comprising the steps of providing, along with suitable reaction components: a) at least one single-strand binding protein, at least one, and preferably at least two target sequence specific primers, at least one DNA polymerase, and optionally: providing at least one reverse transcriptase; b) providing at least one biological sample to be analyzed for the presence of the at least one target sequence, and optionally providing a preferably sterile extraction kit to obtain the biological sample; c) optionally: providing at least one probe and optionally providing at least one enzyme activating the probe so that a detectable signal is generated; d) initiating the amplification reaction by adding at least one starting reagent and/or by contacting the components of (a), (b) and optionally (c); and e) obtaining the at least one amplified target sequence and/or obtaining at least one detectable signal each signal being indicative of a successful amplification of the respective target sequence amplified.

The DNA polymerase used in any aspect of the present invention is preferably a DNA polymerase having a strand displacing activity, i.e. in the polymerization reaction it is able to displace a DNA strand that is hybridized or partially hybridized to the DNA strand on which the polymerase is performing the polymerization reaction.

A "biological sample" is to be understood broadly and relates to any material isolated from a living organism, which contains genetic material from this organism and/or (additionally) from a pathogen (a pathogenic prokaryote or eukaryote) or virus (with an RNA or a DNA genome, single, or double-stranded, or a retrovirus) having infected or infiltrated the organism, or living in symbiosis with this organism etc. A biological sample includes, for example, swab and saliva samples, including nasopharyngeal and oropharyngeal swab or saliva, but also sputum, blood, urine and stool and the like. Preferably, a biological sample to be tested in a non-laboratory environment will be a swab, saliva, urine, or stool sample etc. that can be retrieved without the assistance of trained medical personnel.

Suitable reaction components for enzymatic reactions and the concentration and compounding thereof, in case the enzyme and the reaction for which these suitable reaction components are intended is known, can easily be determined by the skilled person. Certain aspects of particular relevance for the methods of the present invention will be disclosed in detail in the following.

A "starting reagent" as used herein according to the various methods, can be particularly suitable, as this reagent allows an exact and controllable start of the reaction. A starting reagent can be an essential co-factor of at least one enzyme without which the enzymatic reaction cannot start. For example, the starting reagent may be magnesium, or a salt thereof or a magnesium containing solution, for example, magnesium acetate. The concentration can vary and may be between approx. 5 nM and 20 mM. The starting reagent may be provided in an activatable form, in a separate compartment, or in a heat activatable manner so that the start of the reaction can be tightly controlled.

Further, a nucleic acid amplification reaction will require dNTPs (nucleotide triphosphates), for example, dATP, dGTP, dCTP, and dTTP. In leading and lagging strand amplifications, ATP, GTP, CTP, and UTP may also be included for synthesis of RNA primers. In addition, ddNTPs (ddATP, ddTTP, ddGTP and ddGTP) may be used to generate fragment ladders. The dNTPs may be used at a concentration of between 1 µM to 200 mM of each NTP species. A mixture of dNTP and ddNTP may be used with ddNTP concentrations at 1/100 to 1/1000 of that of the dNTP (1µM to 200 mM). The use of UTP may be particularly suitable in case a carry-over prevention kit (e.g., Thermo Fisher using a so-called AmpErase) is used. In these embodiments, a suitable, usually recombinant, uracil N-glycosylase (UNG) can be used to prevent that an amplification product is again amplified in a subsequent reaction. To this end, the reaction can perform faster and false-positive results can be avoided.

Further, a reducing agent may be used, wherein the reducing agents to be used includes dithiothreitol (DTT). The DTT concentration may be between 1 mM and 50 mM.

The buffer solution in an isothermal method of the present invention may be a Tris-HCI buffer, a Tris-Acetate buffer, or a combination thereof, but other buffer systems are suitable as well. The buffers may be present at a concentration of between around 10 nm to 500 mM, which will depend on the buffer system used and the pH of the reaction to be achieved. The latter is influenced by the optimum activity of an enzyme of interest, as it is known to the skilled person.

Standard suitable reaction conditions and concentrations of agents for the isothermal methods as disclosed herein (e.g., enzymes, primers, probe, dNTPs, ATP, buffers, pH, etc.) are known to the skilled person and cancan be taken from, for example, Zanoli and Spoto, *supra,* Li et al., *supra,* Hoser et al., *supra,* Van Ness et al, *supra,* whereas special conditions to be taken into consideration are disclosed herein for special embodiments.

For the purpose of the present invention, a short amplicon length will usually be preferable, as it increases the efficiency of the amplification. Further, the shorter the target to be amplified (and thus the shorter the amplicon), the faster the results of the cognate test will be available. Of course, this will represent a huge advantage for rapid tests performed, for example, at home to have rapid certainty on a possible infection.

The design of primers and optionally a probe will be of particular importance when the methods as disclosed herein are performed to provide reliable diagnostic results. In preferred embodiments according to the methods of the present invention, particularly if used for diagnostic purposes, the general amplicon length, which can be influenced by the corresponding primer design, will usually range from about 50 to about 400 base pairs (bp), preferably 100 to about 200 bp, and most preferably about 120 to about 150 bp. An amplicon length below 200 bp is considered to be favorable for diagnostic purposes as the length of the amplicon has an influence on the efficiency of the amplicon amplification and, therefore, is directly linked to the analytical sensitivity of the reaction.

Unlike for PCR, primers in context of the present invention are usually relatively long (between about 30 and 35 nucleotides). Shorter PCR primers (between 18 and 25 nucleotides) can also be used in the present invention but may decrease the reaction speed and sensitivity, which is to be avoided in diagnostic settings.

Isothermal amplification was initially demonstrated to be a nucleic acid amplification method for DNA. It was later also shown to be suitable for RNA amplification if an additional enzyme, a reverse transcriptase (e.g., a Murine Leukemia virus (MuLV) reverse transcriptase) is added to the same reaction compartment. In the context of viral diagnostics, the RNA amplification and thereby detection of viral RNA is immensely important. It does not only allow the detection of genetic material from RNA viruses, it further allows the detection of viral transcripts also from DNA viruses. The latter can be of great importance, for example, in such cases where viral transcripts are released into the bloodstream while the virus particles and the viral DNA mostly remain within a tissue from which a biological sample cannot easily be provided.

However, the addition of another enzyme, the reverse transcriptase, increases the complexity and costs of the diagnostic tool. Therefore, in the context of the present invention, which aims to provide cheap testing tools with reduced complexity, it is of particular interest to provide one protein that comprises both the functionality of a DNA polymerase and a reverse transcriptase.

A reverse transcriptase may be any enzyme that can generate a complementary DNA (cDNA) strand from a template RNA molecule. "Functionality" in the context of the present invention refers to the ability perform the function of a certain enzyme in light of the present invention. Thus, a reverse transcriptase functionality refers to the ability of to generate cDNA from a target RNA in the reaction mix and DNA polymerase functionality refers to the ability to polymerize DNA in on a target DNA strand in the reaction mix.

In one embodiment according to the first aspect of the present invention, at least one reverse transcriptase is provided, preferably wherein the functionality of the at least one reverse transcriptase and the functionality of the at least one DNA polymerase are provided as a single protein.

A single protein as used herein refers to a protein that consists of one continuous polypeptide chain, or two or more subunits that interact closely, such as a homo- or hetero-dimer, wherein the single protein can have more than one distinct function.

In certain embodiments, the methods of the present invention may be used for a target sequence that is a single stranded or double stranded DNA, cDNA and/or RNA sequence. In turn, the method can be used for all kinds of amplification reactions on all kinds of nucleic acid material.

In one embodiment according to the first aspect of the present invention, the target sequence is a single stranded or double stranded DNA and/or RNA nucleic acid sequence.

In a preferred embodiment according to the first aspect of the present invention, DNA and RNA nucleic acid sequences as target sequences are provided. For the detection of DNA viruses or retroviruses the combined detection of both viral DNA and viral RNA (by reverse transcription in the same procedure) offers the great advantage that the sensitivity is strongly increased as all possible targets that may be present in a sample, such as the viral DNA itself and additionally the RNA transcripts of the viral DNA, can be detected together.

In certain embodiments, the methods according to all aspect of the present invention use both a reverse transcriptase activity as defined above, preferable wherein the functionality of a reverse transcriptase and the functionality of a DNA polymerase are provided on the same protein having dual functionality, to provide a method that achieves fast and reliable polymerization results while having a reduced complexity. This makes the use of the dual functionality enzyme a preferred option for maintaining a high amplification rate and quality, whilst drastically reducing the complexity of the assay components. Reducing the complexity of the assay with regard to the protein/enzyme components used will significantly reduce costs and susceptibility to deleterious interference caused by use of a high number of diverse enzymes, wherein each enzyme has its own requirements to be functional and a smooth interplay of enzymatic functions in one and the same assay or reaction is not always easy.

In certain embodiments, the reaction mix may further include RNase inhibitors.

In one embodiment according to the first aspect of the present invention, the method is conducted at a temperature of about 20°C to about 50°C, preferably of about 22°C to about 45°C, and most preferably from about 25°C to about 42°C.

In certain embodiments, at least one or preferably all enzymes of the method of the present invention are thermostable enzymes. A "highly thermostable enzyme" as used herein can be characterized by showing denaturation only at temperatures above around 70°C, 71 °C, 72°C, 73°C, 74°C and preferably above 75°C and/or an optimum activity temperature at 37°C to about 85°C, preferably of about 38°C to about 80°C, and most preferably from about 40°C to about 65°C. The advantage of thermostable enzymes is the fact that these enzymes will have optimum activity, meaning optimum catalytic activity, above a standard (body) temperature of 37°C. An increased temperature increases the interaction rate of the reagents with one another and, thus improves the reaction speed. Thus, an increased temperature is used in certain embodiments according to all aspects of the present invention to provide a method as defined in the first aspect of the present invention that has a reaction mix with a reduced complexity but still achieves reliable polymerization results.

In certain embodiments, the methods according to all aspect of the present invention use both a reverse transcriptase activity as defined above, preferable wherein the functionality of a reverse transcriptase and the functionality of a DNA polymerase are provided on the same protein, and increased temperatures to provide a method that achieves reliable polymerization results while having a reduced complexity.

In one embodiment of the methods of the present invention, at least one forward and at least one reverse primer are provided as target sequence specific primers, wherein at least one of these primers, preferably the reverse primer, is provided in a higher amount or concentration in relation to the other primer. Usually, primers, or a set of primers, or several sets of primers, of a multiplex analysis is of interest, can be provided according to the methods as disclosed herein, wherein the primers are usually provided in an equimolar ration. Still, it was found out that for certain purposes, e.g., for diagnostic of an RNA virus, it may be suitable to provide a higher amount or concentration of one primer (depending on the fact whether it is a (+) or (-) ssRNA virus) to obtain more cDNA for the following amplification step, which will increase the performance of the reaction.

In one embodiment according to the first aspect of the present invention, at least one forward and at least one reverse primer are provided as target sequence specific primers, wherein at least one of these primers, preferably the reverse primer, is provided in a higher amount or concentration in relation to the other primer.

In certain embodiments, a probe may be used in the methods as disclosed herein, wherein preferably at least two different probes are used: one associated with the target nucleic acid to be amplified, and one associated with a positive control. The use of reliable positive controls, particularly in the field of diagnosis, more particularly the diagnosis of infections agents like SARS-CoV-2, is very helpful and not included in presently available test kits, as the positive control allows a conclusion whether the reaction as such worked. This is of particular importance and a huge advantage in case the test is not performed by trained experts in a laboratory but rather in a home environment or in test stations.

The term "probe" as used herein broadly refers to a molecule or atom used in molecular biology, and particularly in the methods as disclosed herein, to study the properties or, for diagnostic setting, the quantity of another molecule or structure of interest. A variety of different probes that can be easily combined with the methods as disclosed herein is available to the skilled person (cf. Molecular Probe Techniques, Tree Number(s) E05.601, Unique ID D015336, RDF Unique Identifier, http://id.nlm.nih.gov/mesh/D015336). For using the methods as disclosed herein on the specific device as disclosed herein, probes, optionally activatable probes, providing a detectable and/or quantifiable signal may be preferred, e.g. Förster/fluorescence resonance energy transfer (FRET) probes etc. In view of the methods as disclosed herein, this may allow a rapid diagnostic result. This offers the advantage to include a positive control associated with one probe, and a different probe associated with the actual target sequence, which may drastically increase the reliability of a diagnostic result.

In the context of the present invention, any luminescent detection system may be used to detect an amplification result of interest fast and reliably. Luminescence is the umbrella term for any kind of spontaneous emission of light by a substance, not resulting from heat. Luminescence comprises various types of luminescence, wherein the most relevant for the purpose of the present invention are chemiluminescence, including bioluminescence and electroluminescence as well as photoluminescence, including fluorescence and phosphorescence.

Probes that can be incorporated during the methods according to the present invention include, for example, a commercially available TwistAmp^{™} exo probe (typically having a length of about between 46 and 52 nucleotides) and the TwistAmp^{®} fpg probe(typically between 32 and 35 nucleotides). These probes are used for fluorogenic real-time detection. These two probes are usually labelled with a fluorophore, a quencher (e.g., a BlackHole Quencher) that is in close proximity to the fluorophore to temporarily deter a fluorescent signal, and a blocker (e.g.C3-spacer, a phosphate, a biotin-TEG or an amine) at the 3'-end serving to prevent polymerase extension from the 3'-end (see again Li et al., Analyst, 2019, *supra,* particularly Fig. 3A and B). The real-time detection is based on cleavage of fluorogenic probes at an abasic site (also known as an apurinic/apyrimidinic site that is a location in DNA (less often in RNA), which has neither a purine nor a pyrimidine base) between the fluorophor and the quencher. The abasic site can either be tetrahydrofuran (THF) or a dSpacer (a derivative of the THF) or a dR group (the deoxyribose of the abasic site via a C-O-C linker). The *E. coli* exonuclease III, for example, cleaves the TwistAmp^{™}exo probe at a THF or a dSpacer site, while the *E. coli* glycosylase/lyase fpg (formamidopyrimidine-DNA glycosylase) cleaves the TwistAmp^{™} fpg probe at the dR position. After the enzymatic cleavage, the TwistAmp^{®} exo probe can serve as a forward primer. However, the TwistAmp^{™} fpg probe cannot serve as a primer due to a different catalytic mode (beta-elimination) of the *E. coli* glycosylase/lyase fpg protein, which does not generate an extendable 3'-OH group but a 3'-phosphate group. A variety of different probes or labels for marking a nucleic acid base or a nucleotide of interest with a detectable label, including probes not necessitating the addition of at least one activating enzyme, are imaginable as long as there is at least one suitable light source and a cognate detection device specifically detecting the presence of the probe and/or label by producing a detectable signal.

In another embodiment, a further luminescent luminophore dye may be used (cf. Roy et al., Biosens Bioelectron. 2016 Dec 15; doi: 10.1016/j.bios.2016.06.065.).

As it is known in the field of molecular detection techniques, comprising the use of PCR and of isothermal methods, there are various ways of visualizing the result of an amplification reaction. These techniques are known to the skilled person and can be used for the methods as disclosed herein.

In one embodiment, for example, the at least one primer, including single-stranded and partially double-stranded primers, is itself labelled with a detectable label. It should be noted that a fluorescence quencher is also considered a detectable label. For example, the fluorescence quencher may be contacted to a fluorescent dye and the amount of quenching is detected. Not to interfere with the reaction, the detectable label should be such that it does not interfere with the amplification reaction. Where the primer is partially double-stranded with an invading strand and a non-invading strand, the detectable label should be attached in such a way so it would not interfere with the amplification reaction of the invading strand. The non-invading strand of a partially double-stranded primer is not elongated so there are no limitations on the labelling of the non-invading strand with the sole exception being that the label on the non-invading strand should not interfere with the elongation reaction of the invading strand. Labelled primers offer the advantage of a more rapid detection of amplified product. In addition, the detection of unincorporated label, that is, labelled oligonucleotides that have not been extended, will allow the monitoring of the status of the reaction.

A "label" can be a chemical modification or isotope substitution that enables the identification of a molecule, moiety or atom throughout its metabolic transformations or transport, or particularly during the course of a diagnostic or preparative assay of nucleic acid amplification.

In another embodiment, a double stranded primer may be labelled as such that the separation of the two strands of the primer may be detected. As discussed above, after multiple rounds of amplification, the invading strand and the non-invading strand of a partially double stranded primer are separated. After this separation, the non-invading strand does not participate in the amplification reaction, which in turn may be used to detect and monitor the result of the amplification reaction on-line.

Further, the detectable label may be a fluorescent label or an enzyme and the label quencher (also referred to as the label inhibitor) may be a fluorescence quencher or an enzyme inhibitor. In these cases, the label is detected by fluorescence or enzyme inhibition. The delectability of the label would be the fluorescence if a fluorescent label is used or enzyme activity if an enzyme is used.

In a preferred embodiment, a separate probe is provided, which may be target sequence specific. Further, the probe may serve the purpose to provide a detectable or visible signal to monitor the result of the amplification reaction in a convenient manner. The probe may carry an activatable label or signal-giving component, which can be activated, for example, by the addition of an enzyme, for example an exonuclease III (Exo III, exo herein) as used in Twist-RPA reactions. The label may also be associated with at least one primer, in certain embodiments. Even though the detectable moiety may be selected from the group consisting of a fluorochrome, an enzyme, a fluorescence quencher, an enzyme inhibitor, a radioactive label, a chromogenic agent, and a combination thereof, for a non-laboratory use of the methods of the present invention, the use of radioactive material and/or generally the use of hazardous substances (of chemical, biological or other nature) should be avoided to allow a fast regulation and safety for the end user of the system, particularly if used in a private environment. Further, this may be favorable for waste management, as the system when used can be easily discarded.

In one embodiment according to the first aspect of the present invention, at least one probe and/or label is provided, wherein the at least one probe and/or the at least one label comprise a directly or indirectly detectable moiety.

All primers or probes as used in the methods as disclosed herein can be naturally occurring DNA and/or RNA sequences, or synthetic primers or probes including naturally occurring bases and/or backbones, or synthetic elements including labels covalently and/or non-covalently attached to the primer or probe, or including at least one phosphothioate backbone for increasing the stability of the primer or probe, or to improve the amplification of DNA sequences by DNA polymerases with proofreading activity (Skerra, Nucleic Acids Res 20, 3551-3554, doi:10.1093/nar/20.14.3551 (1992)), and any combination thereof.

In one embodiment according to the first aspect of the present invention, the at least one single-strand binding (SSB) protein originates from Escherichia phage RB69, preferably wherein the single-strand binding protein has a sequence disclosed as reference sequence NP_861936.1, or a sequence having at least 90%, 91%, 92%, 93%, 94%, 95%, 96%, 97%, 98%, or 99% identity thereto, or a functional analogue of this sequence. The sequence has been made publicly available as accession number NP_861936, preferably version NP_861936.1, as NCBI Reference Sequence (database National Center for Biotechnology Information (NCBI); see https://www.ncbi.nlm.nih.gov/) making reference to the cognate RB69 phage genome made publicly available as NC_004928.1 (also at NCBI).

A single-strand binding protein (also called single-stranded (DNA-)binding protein) as used according to any aspect of the present invention, relates to proteins of the class of single-strand binding protein (e.g. reviewed in James L. Keck (ed.), Single-Stranded DNA Binding Proteins: Methods and Protocols, Methods in Molecular Biology, vol. 922, DOI 10.1007/978-1-62703-032-8_1, # Springer Science+Business Media, LLC 2012).

In certain embodiments, a single-strand binding protein according to the various aspects of the present invention may be a phage derived single-strand binding protein.

In a preferred embodiment, the single-strand binding protein may be a protein originating from the phage RB69 (NP_861936.1, *supra*)*.* This protein binds preferentially to single-stranded DNA and, therefore, destabilizes double-stranded DNA. As its natural function, it binds single-stranded DNA as the replication fork advances and stimulates the replisome processivity and accuracy, which can of course be exploited in the PINA reaction as disclosed herein, as the protein has excellent stabilization effects. Further, the present inventors could demonstrate that the use of this specific single-stranded DNA protein allows both (i) establishing an effective PINA reaction not needing any recombinase and thus drastically simplifying the reaction, and (ii) allowing to amplify all kinds of single-stranded or double-stranded DNA or RNA, be it in a circular or linear(ized) form.

The at least one SSB protein as used according to any embodiment of the present invention may be used in a concentration between 50 ng/µl and 1500 ng/µl, preferably between 100 ng/µl and 800 ng/µl, more preferably between 200 ng/µl and 600 ng/µl and most preferably between 300 ng/µl and 500 ng/µl.

In one embodiment according to the first aspect of the present invention, any of the proteins or enzymes used comprises at least one tag and/or label.

Protein tags, i.e., artificial sequences added to the coding sequence of a protein or enzyme of interest, are well known to the skilled person in the relevant technical field of biotechnology and molecular biology. These tags are added to a protein to facilitate the purification after the recombinant production thereof (so-called affinity tag), the visualization, the solubility of a protein etc.

According to the various embodiments of the present invention, every enzyme used according to the methods as claimed and disclosed herein may comprise at least one tag, either at the N-terminus, or at the C-terminus, or also within the protein sequence (without disturbing the function of the protein, preferably between to individual domains), or any combination thereof (e.g., N- terminal and C-terminal tag, which will usually be different, e.g. two different affinity tags, or the combination of a solubility tag and a visualization tag etc.). The term "tag" or "protein tag" thus refers to a variety of different polypeptide sequences or the corresponding nucleic acid sequences encoding the same, which can be incorporated into a protein of interest at various positions, preferably at the N- and/or C-terminus, or between distinct functional domains, and fulfil several different functions. Protein tags can be, for example, affinity tags, such as chitin binding protein (CBP), maltose binding protein (MBP), Strep-tag and glutathione-S-transferase (GST) or His-Tag. Affinity tags can be used for specifically purifying the protein of interest. Another example of protein tags are solubilization tags, which allow to obtain the protein of interest in its soluble form especially in bacterial cell culture. Epitope tags can be used for having a binding site for analytical antibodies. Luminescence, preferably fluorescence tags offer the possibility to detect the protein of interest in the cell culture. A tag or label can also serve the purpose of labelling a protein or enzyme of interest, or a fragment thereof, to allow an easy identification. This may be of particular interest for diagnostic purposes.

In a preferred embodiment, no additional crowding agent has to be used, as the reduction of protein/enzyme interaction partners with the PINA methods suggested herein obviates the need of additional crowding agents.

A "crowding agent" as used herein is meant to specify any agent assisting in providing an optimum reaction environment for an enzymatic reaction. The mode of action of a crowding agent is the creation of smaller reaction compartments so that the probability of a reaction between a substrate and the cognate enzyme is increased. Crowding agents usually actively added to isothermal amplification reactions include, for example, polyethylene glycol (PEG), dextran and ficoll. The crowding agent may usually be at a concentration between 1% to 12% by volume (v/v) or 1% to 12% by weight (w/v) of the reaction. While all PEG polymers are useful, preferred PEG compounds were disclosed to include PEG1450, PEG3000, PEG8000, PEG10000, PEG compound molecular weight 15000-to 20,000 (also known as Carbowax 20M), and a combination thereof.

Alternatively or additionally, nanostructured elements may be used in the reaction mixtures of the present invention to further optimize the amplification result, for example, ZnO nanostructures (Ma et al., Virus Res., 2017, 232: 34-40. doi: 10.1016/j.virusres.2017.01.021.).

A reaction according to the present invention may be incubated between 5 minutes and 16 hours, such as between 15 minutes and 3 hours or between 30 minutes and 2 hours. The incubation may be performed until a desired degree of amplification is achieved (cf. EP 2 336 361 A2). In certain embodiments, the reaction may be performed under agitation to increase the contact rate of the various components and, thus, the efficacy of the method.

In one embodiment according to second aspect the present invention, there may be provided a kit for performing a method according to the first aspect of the present invention, wherein the kit comprises (i) at least one single-strand binding protein, at least one DNA polymerase, and optionally: at least one reverse transcriptase, together with suitable reaction components, (ii) at least one, preferably at least two target sequence specific primers; (iii) suitable reaction components, including at least one co-factor for at least one enzyme, dNTPs or a mixture of dNTPs and ddNTPs, at least one buffer system, and at least one reducing agent; (iv) at least one sterile set for extracting a biological probe to be analyzed and comprising at least one component configured to be applied to a system for analyzing the probe (such a system is disclosed in European patent application EP20201885.9); (v) optionally: at least one probe and optionally at least one enzyme activating the probe; and (vi) optionally: wherein the kit further comprises a second part comprising (i) to (v), wherein the second part comprises a predetermined second target sequence acting as positive control and at least one, preferably at least two primers specific for the second target sequence, and preferably a second probe. Optionally, the kit can further comprise instructions for use.

It may be highly suitable that the kit comprises optimized primers to detect the genetic material of an agent, usually an infectious agent, as target sequence in a highly specific manner. Further, it will be useful to provide a sterile set, including gloves, to extract a biological sample in a preferably non-invasive manner.

In a laboratory, the skilled molecular biologist or trained personnel can easily conduct the methods as disclosed herein, design primers and/or probes for target nucleic acids to be amplified, and suitable reaction conditions can be easily modified, if desired. Of course, it is a much bigger challenge to perform the method in a non-laboratory environment. Particularly in the latter situation, it may be favorable to provide a kit comprising all necessary components in ready-made sterile vials or tubes, so that the (bio-)chemical agents and components can easily be re-activated (for enzymes, for example, after freeze-drying) and the reaction can be started in a convenient manner.

In one embodiment according to the third aspect of the present invention, there may be provided the use of a kit as defined in the second aspect of the present invention, for performing an isothermal method of nucleic acid amplification of at least one target sequence as defined in the first aspect of the present invention.

As is detailed for the methods of the present invention, these methods have been designed in a way that the complexity is reduced, i.e. fewer enzymes and chemical agents are needed than for known isothermal amplification methods. This simplicity and reduced complexity of the method has the advantage that the various parts of the kit can be reduced to make it cheaper, and easier to use in a home environment.

The present invention will now be further illustrated with reference to the following nonlimiting Examples.

### Examples

### Example 1 (reference example): Establishing an RT-RPA for SARS-CoV-2

Initially, a real-time reverse transcription RPA (RT-RPA) targeting the RNA-dependent RNA polymerase (RdRP), envelope protein (E) and nucleocapsid (N) genes of SARS-CoV-2 was established to determine the limit of detection, cross reactivity and clinical performance in comparison to real-time RT-PCR (Abd EI Wahed et al., 2021, Suitcase lab for rapid detection of SARS-CoV-2 based on recombinase polymerase amplification assay, Anal. Chem. 2021, 93, 4, 2627-2634).

For assay validation, molecular RNA standards based on the RdRP -, E- and N-gene (Accession number: NC_045512, nucleotides: 14977-15975, 26112-26479 and 28280-29536, respectively) were synthesized by GenExpress (Berlin, Germany). Oligonucleotides for the RdRP and E genes were updated from our previous design for SARS-CoV-1 and MERS CoV (unpuplished data), whereas the N gene amplicon was modified from a previously published article (Behrmann, O. et al. Rapid detection of SARS-CoV-2 by low volume real-time single tube reverse transcription recombinase polymerase amplification using an exo probe with an internally linked quencher (exo-IQ). Clin Chem, doi:10.1093/clinchem/hvaa116 (2020)). **(Table 1).** All oligonucleotides were synthesized by TIB MOLBIOL GmbH (Berlin, Germany).

**Table 1. RT-RPA assay oligonucleotides:**

| **Gene** | **Oligonucl eotide** | **Sequence 5'-3'** |
|---|---|---|
| RdRP | Forward | TATGCCATTAGTGCAAAGAATAGAGCTCGCAC (SEQ ID NO: x) |
| | Reverse | CAACCACCATAGAATTTGCTTGTTCCAATTAC |
| | Exo-Probe | TCCTCTAGTGGCGGCTATTGATTTCAATAAbTXfTTTGATGA AACTGTCTATTG-PH |
| E | Forward | GAAGAGACAGGTACGTTAATAGTTAATAGCGTA |
| | Reverse | AAAAAGAAGGTTTTACAAGACTCACGTTAACsA |
| | Exo-Probe | ATCGAAGCGCAGTAAGGATGGCTAGbTXfTAACTAGCAAGA ATAC-PH |
| N | Forward | CCTCTTCTCGTTCCTCATCACGTAGTCGCAAC |
| | Reverse | AGTGACAGTTTGGCCTTGTTGTTGTTGGCCTT |
| | Exo-Probe | TAGAATGGCTGGCAATGGCGGTGATGCTGCbTXfTTGCTTT GCTGCTGCTT-PH |

| | | |
|---|---|---|
| BHQ1-dt (bT), Tetrahydrofuran (X), Fam-dT (fT), phosphothioate backbone (s) and PH: 3' phosphate to block elongation. | | |

### Example 2: Testing isothermal amplification with reduced complexity

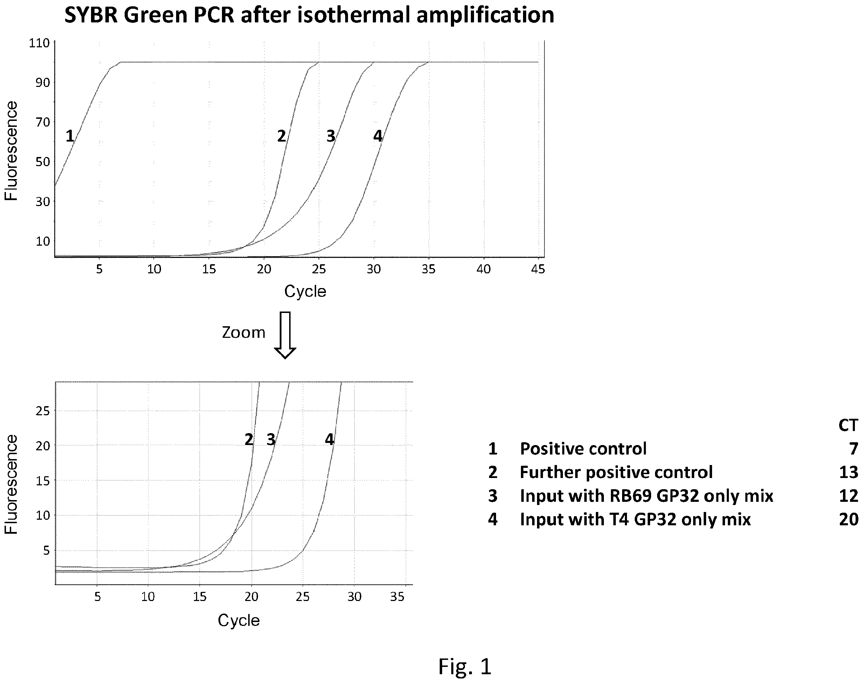

Reaction conditions for PINA assays were tested with SARS-CoV-2 target material as described in Example 1. Visualization of amplified DNA material was performed by a quantitative nested PCR using SYBR Green. Different buffer conditions, different temperatures, primer concentrations and concentrations of the single strand Binding protein were tested. Conditions suitable for yielding good amplification results are shown in **Table 2,** performed at 42 °C and at pH 8.3, and correspond to samples 3 and 4 in **Figure 1****.**

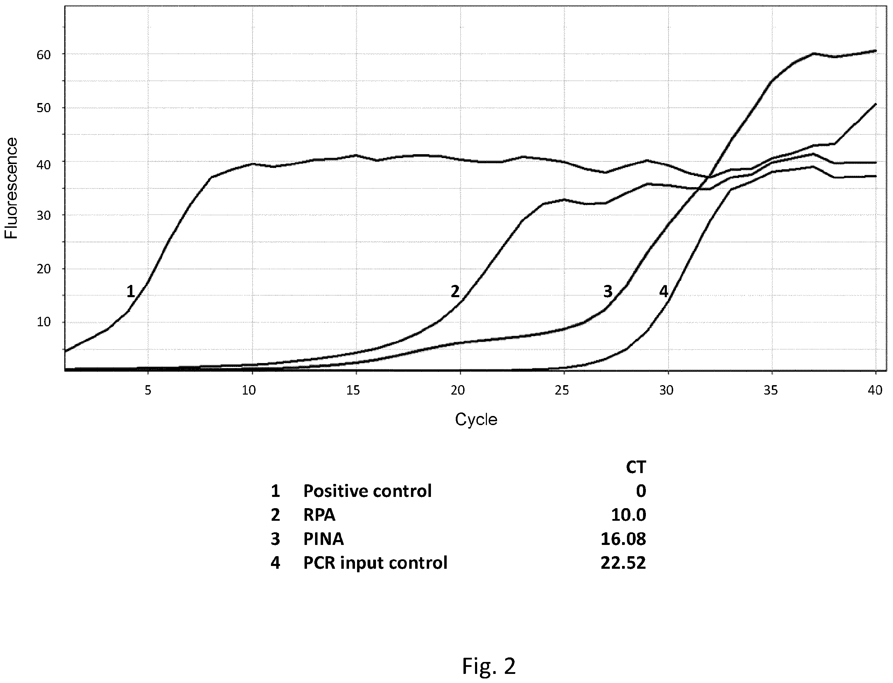

Further, PINA assays where compared to RPA methods containing a recombinase. One experiment using T4 GP32 either in combination with the UsvX recombinase and the UsvY recombinase loading factor (RPA) or in an SSB-only approach (PINA), as detailed in **Table 3,** is shown in **Figure 2****.** Further PINA tests to amplify RNA extracts directly from patient material are ongoing/planned. It was observed that the conditions of **Table 1** may be suitable for amplification of RNA from patient material, however, the different concentrations can be adapted with the above guidance, because the concentration of viral material in patient samples is lower.

Our work thus demonstrate that the PINA assay works, for both RNA standard material and patient material, and needs a much less complexity of the enzyme/protein mix used. This can be a huge advantage for home tests.

**Table 2. Ingredients of PINA assay**

| | **Ingredients** | **Final concentration** |
|---|---|---|
| Buffer | TRIS Base, for example Trizma | 350 mM |
| | Potassium acetate | 100 mM |
| | DTT | 20 mM |
| | dNTP Mix | 2 mM |
| | ATP* | 30 mM (optional) |
| Oligomix Sars-CoV2 RdRP gene | Forward Primer | 420 nM |
| | Reverse Primer | 840 nM |
| | Probe | 240 nM (optional) |
| SSB protein: T4 GP32 or RB69 GP32 | | 600 ng/µl |
| Creatine kinase* | | 100 ng/µl (optional) |
| Phosphocreatine* | | 50 mM (optional) |
| PEG | | 5 % (optional) |
| *Bsu* DNA polymerase | | 30 ng/µl |
| Exonuclease | | 80 ng/µl (optional) |
| Mg(OAc)₂ | | 14 mM |

| | | |
|---|---|---|
| * ATP and the energy regeneration components creatine kinase and phosphocreatine are not required for PINA assays but only for RPA based control reactions. | | |

**Table 3. Reaction conditions of PRA and PINA**

| | UvsX [ng/µl] | UvsY [ng/µl] | T4gp32 [ng/µl] | Creatine kinase [ng/µl] | *Bsu* DNA polymerase [ng/µl] | RPA Time [minutes] |
|---|---|---|---|---|---|---|
| 1 | Positiv Control | | | | | 15 |
| 2 | 60 | 15 | 1500 | 25 | 7,5 | 15 |
| 3 | - | - | 1500 | 25 | 7,5 | 15 |
| 4 | PCR Input Control | | | | | |

### Example 3: Reverse transcription and isothermal amplification

The basic principle and operability of PINA-based amplification is described in example 2. Further we intend to show the PINA assay in combination with a reverse transcriptase and, especially, in an approach that uses an enzyme with both DNA polymerase and reverse transcriptase activity in one protein. Such an enzyme is preferably one that may be designed by directed *in vitro* evolution. These analyses will use RNA target material as described in the previous examples but without any prior reverse transcription. The test settings will include different reverse transcriptase enzymes, separately or as a dual-function polymerase and reverse transcriptase single protein. In different test series the enzymes will be tested at different concentrations, with different target RNA concentration and at different temperatures. In each test series designed to address a certain variable parameter, all other parameters are going to be held constant.

## Claims

1. An isothermal method of nucleic acid amplification of at least one target sequence, preferably in a biological sample, the method comprising the steps of providing, along with suitable reaction components:
a) at least one single-strand binding protein, at least one, and preferably at least two target sequence specific primers, at least one DNA polymerase, and optionally: providing at least one reverse transcriptase;
b) providing at least one biological sample to be analyzed for the presence of the at least one target sequence, and optionally providing an extraction kit, preferably a sterile extraction kit, to obtain the biological sample;
c) optionally: providing at least one probe and optionally providing at least one enzyme activating the probe so that a detectable signal is generated;
d) initiating the amplification reaction by adding at least one starting reagent and/or by contacting the components of (a), (b) and optionally (c); and
e) obtaining the at least one amplified target sequence and/or obtaining at least one detectable signal each signal being indicative of a successful amplification of the respective target sequence amplified.

2. The method of claim 1, wherein at least one reverse transcriptase is provided, preferably wherein the functionality of the at least one reverse transcriptase and the functionality of the at least one DNA polymerase are provided as a single protein.

3. The method of any of the preceding claims, wherein the target sequence is a single stranded or double stranded DNA and/or RNA nucleic acid sequence.

4. The method of claim 3, wherein single stranded or double stranded DNA and RNA nucleic acid sequences as target sequences are provided.

5. The method of any of the preceding claims, wherein the method is conducted at a temperature of about 20°C to about 50°C, preferably of about 22°C to about 45°C, and most preferably from about 25°C to about 42°C.

6. The method of any of the preceding claims, wherein at least one forward and at least one reverse primer are provided as target sequence specific primers, wherein at least one of these primers, preferably the reverse primer, is provided in a higher amount or concentration in relation to the other primer.

7. The method of any of the preceding claims, wherein at least one probe and/or label is provided, wherein the at least one probe and/or the at least one label comprise a directly or indirectly detectable moiety.

8. The method of any of the preceding claims, wherein the at least one single-strand binding protein originates from *Escherichia* phage RB69, preferably wherein the single-strand binding protein has a sequence disclosed as reference sequence NP_861936.1, or a sequence having at least 90%, 91%, 92%, 93%, 94%, 95%, 96%, 97%, 98%, or 99% identity thereto, or a functional analogue of this sequence.

9. The method of any of the preceding claims, wherein any of the proteins or enzymes used comprises at least one tag and/or label.

10. A kit for performing a method of any one of claims 1 to 9, wherein the kit comprises
(i) at least one single-strand binding protein, at least one DNA polymerase, and optionally: at least one reverse transcriptase, together with suitable reaction components,
(ii) at least one, preferably at least two target sequence specific primers;
(iii) suitable reaction components, including at least one co-factor for at least one enzyme, dNTPs or a mixture of dNTPs and ddNTPs, at least one buffer system, and at least one reducing agent;
(iv) at least one sterile set for extracting a biological probe to be analyzed and comprising at least one component configured to be applied to a system for analyzing the probe;
(v) optionally: at least one probe and optionally at least one enzyme activating the probe; and
(vi) optionally: wherein the kit further comprises a second part comprising (i) to (v), wherein the second part comprises a predetermined second target sequence acting as positive control and at least one, preferably at least two primers specific for the second target sequence, and preferably a second probe.

11. A use of at least kit as defined in claim 10, for performing an isothermal method of nucleic acid amplification of at least one target sequence as defined in any one of claims 1 to 9.
